# EUROPEAN PATENT APPLICATION

(11) **EP 4 339 277 A1**
(43) Date of publication of application: **20.03.2024**
(21) Application number: 23191658.6
(22) Date of filing: 16.08.2023
(51) Int. Cl.: C12M 1/34, C12M 1/36

(54) **CELL CULTURE SYSTEM AND CELL CULTURE METHOD**

(30) Priority: 24.08.2022 JP 2022133288
(71) Applicant: Yokogawa Electric Corporation, Tokyo 180-8750 (JP)
(72) Inventor: NAMATAME, Tetsushi, Musashino-shi, Tokyo, 180-8750 (JP); NAKAMURA, Yukihiro, Musashino-shi, Tokyo, 180-8750 (JP); KURIHARA, Nobuto, Musashino-shi, Tokyo, 180-8750 (JP); SAKAKI, Ayumu, Musashino-shi, Tokyo, 180-8750 (JP)
(74) Representative: Winter, Brandl - Partnerschaft mbB

(57) **Abstract**

Stable operations can be performed even when a variation occurs between batches in perfusion culture. A cell culture system includes a culture tank for culturing cells, an information processing system that processes various pieces of information on the culture of the cells in the culture tank, and a controller that controls a culture step by the culture tank. The information processing system stores an operation condition in each of a plurality of steps constituting the culture step of the cells, stores a step transition condition for transition to a next step, and sets an operation condition for the next step when the step transition condition is satisfied during performing one step.

## Description

### BACKGROUND

### Technical Field

The present disclosure relates to a cell culture system and a cell culture method.

### Background Art

Production of bio-pharmaceuticals includes a culture step of producing a target substance via biological reactions and a purification step of removing contaminants generated in the culture step to increase a degree of purity of the target substance. In the culture step of antibody pharmaceuticals representing the bio-pharmaceuticals, mainly animal cells, such as CHO cells, have often been used. However, the animal cells are susceptible to a culture environment, and when the environment is not appropriately maintained, this affects the yield and the quality of target products.

Examples of factors that deteriorate the culture environment include mechanical stress caused by stirring or gas aeration, depletion of nutrients or oxygen, and accumulation of waste products, such as lactic acid or ammonia, generated by cells. Accordingly, a production method in which substances required by cells are supplemented during culture while fundamental environmental factors, such as the dissolved oxygen (DO) level, pH, temperature, and a stirring speed in a culture fluid, are controlled is employed. Substances supplemented during culture include nutrients contained in a medium and substances that improve the cell growth speed or production speed referred to as potentiators.

The culture method that supplements the substance in the culture includes a Continuous Culture, a Perfusion Culture, and a Fed-Bach Culture. The continuous culture is a method that removes a part of a culture fluid containing cells in the culture and additionally introduces a culture fluid equivalent to the removed amount for culture.

The perfusion culture is a method that selectively removes a part of a liquid component after cell separation during culture and additionally introduces a culture fluid equivalent to the removed amount for culture. Especially, in the production of antibody pharmaceuticals, since the perfusion culture easily maintains cells at a high density, the production speed per hour can be kept high, and many study reports have been made.

At the start of culture by the perfusion culture, a medium and cells are put in a culture tank, and cell growth progresses by appropriately managing the temperature, the pH, and the dissolved oxygen (DO) level of the culture tank. At the phase where the cells grow to some extent (two days to three days), the medium is continuously supplied to the culture tank, and a cell removal solution (harvest solution) by the same amount as the supplied amount is collected. Since the supplied amount and the collected amount are the same amount, the culture fluid volume in the culture tank is kept constant. Additionally, since the cells remain in the culture tank by a filter action of a cell removal device referred to as tangential flow filtration (TFF) or alternating tangential flow filtration (ATF), even after perfusion starts, the growth continues in the culture tank.

When the number of cells in the culture tank grows to a certain density or more, controlling the dissolved oxygen (DO) level described above and adjusting the nutrient become difficult, leading to deterioration of the culture environment. Therefore, generally, to reduce the cell density in an appropriate range, means (bleed) that discharges the amount of cells excessively increased from the time point of the growth exceeding the target value to outside the culture tank by removing the culture fluid is taken.

Usually, a perfusion rate (a rate of an amount of introduction of a medium to a culture fluid volume per day) is around from 1 to 3 VVD, an amount of bleed liquid to be discharged is around from 0.2 to 0.5 VVD, and the operation is performed around from 4 weeks to 12 weeks. Moreover, glucose as the main nutrient is kept at an appropriate concentration by additionally feeding glucose by the shortage.

Thus, in perfusion culture, in addition to the temperature, the pH, the dissolved oxygen (DO) level, and the like:
(i) amount of supplied medium;
(ii) amount of culture fluid;
(iii) cell density;
(iv) glucose concentration;
and the like also need to be controlled. Since each of them has an appropriate control range depending on cell species, control thereof is not easy. In a case where the control is not appropriately performed, for example, the yield decreases due to a decreased cell activity, and additional operations for recovery therefrom is required. In the worst case, the batch loss occurs, and there may be a case where the cultured cells need to be wasted.

In the substance production using the cell culture, scale-up is performed up to the capacity at which culture is finally performed in order to secure the amount of production. The cells can have a high density in the perfusion culture, the production at a small capacity is possible. However, scale-up of several phases from at the time point of cytolysis (several tens of mL), condition examination scale (to several L), to production scale (to several hundreds of L) is necessary. That is, at the time point where the culture for a desired scale starts, cells on which some manual culture operations (such as freezing, melting, and flask culture) have already been performed in the previous steps. Thus, the cells affected by them are used for the scale-up.

In the plurality of phases in the scale-up, the culture cannot be always started with the cells in completely the same state. Furthermore, the adjustment of the cell density at the time point of starting the culture is based on the result of the cell count including a certain amount of error (around ±10%), and therefore the initial seeding density varies. Since the culture starts in such a situation, various parameters for cell growth and other culture parameters vary to some extent between batches in the parameters for cell growth and other culture parameters. It is not easy to suppress the variation between the respective batches derived from an organism process. WO2020/252442 and US2019/0153381 are exemplified as a prior art.

### SUMMARY

The present disclosure provides a cell culture system and a cell culture method that allow performing stable operations even when a variation occurs between batches in perfusion culture.

A cell culture system according to the present disclosure comprises a culture tank, an information processing system, and a controller. The culture tank is for culturing cells. The information processing system processes various pieces of information on the culture of the cells in the culture tank. The controller controls a culture step by the culture tank. The information processing system stores an operation condition in each of a plurality of steps constituting the culture step of the cells, stores a step transition condition for transition to a next step, and sets an operation condition for the next step when the step transition condition is satisfied during performing one step.

A cell culture method according to the present disclosure in a cell culture system including a culture tank for culturing cells comprises: storing an operation condition in each of a plurality of steps constituting a culture step of the cells and storing a step transition condition for transition to a next step; and setting an operation condition for the next step when the step transition condition is satisfied during performing one step.

The cell culture system and the method according to the present disclosure allow performing stable operations even when a variation occurs between batches in perfusion culture.

### BRIEF DESCRIPTION OF THE DRAWINGS

FIG. 1 is a schematic diagram describing an overall configuration of a cell culture system 1 according to an embodiment;
FIG. 2 is a schematic diagram describing details of an information processing system 20;
FIG. 3 is a flowchart depicting operations of the cell culture system 1 of an embodiment;
FIG. 4 is a flowchart depicting operations of the cell culture system 1 of the embodiment;
FIG. 5A to FIG. 5E are graphs describing an example of step transition conditions;
FIG. 6A to FIG. 6D are graphs describing an effect of this embodiment;
FIG. 7A to FIG. 7D are graphs describing an effect of this embodiment; and
FIG. 8 is a graph describing an effect of this embodiment.

### DETAILED DESCRIPTION

The following describes these embodiments with reference to the attached drawings. In the attached drawings, functionally the same components may be represented with the same number. Note that while the attached drawings illustrate embodiments and exemplary implementations according to the principle of the disclosure, these are for understanding the disclosure, and are never used for interpreting the disclosure in a limited way. Description in this description is merely a typical example, and is not to limit claims or application examples of the disclosure in any sense. While in these embodiments, its description is made in detail enough for a person skilled in the art to implement the disclosure, other implementations and configurations are also possible, and it should be understood that changes in configurations and structures and replacement of various components are allowed without departing from a scope or a spirit of the technical idea of the disclosure. Accordingly, the subsequent description should not be interpreted in a limiting sense.

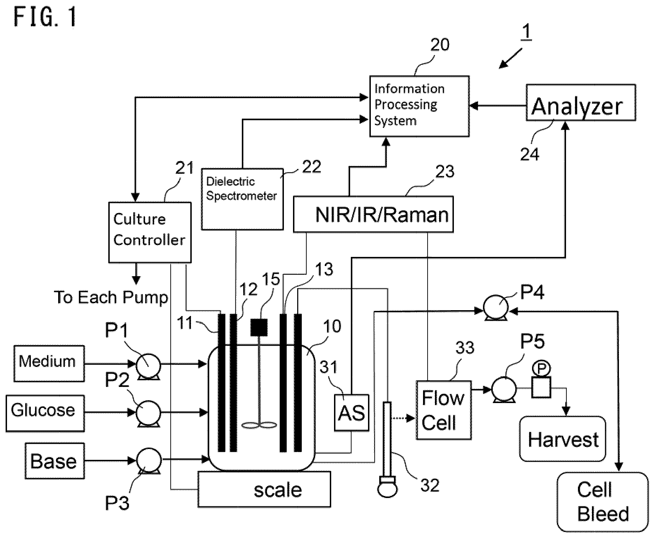

With reference to FIG. 1, an overall configuration of a cell culture system 1 according to the embodiments is described. The cell culture system 1 includes a culture tank 10 that internally stores a culture fluid and an information processing system 20 that processes various pieces of information on culture of cells in the culture tank 10. The system 1 is a system for culturing cells through application of perfusion culture. The system 1 further includes, for example, a culture controller 21, a dielectric spectrometer 22, a near-infrared/infrared/Raman spectrometer 23, an analyzer 24, a cell removal device 32, and a flow cell 33.

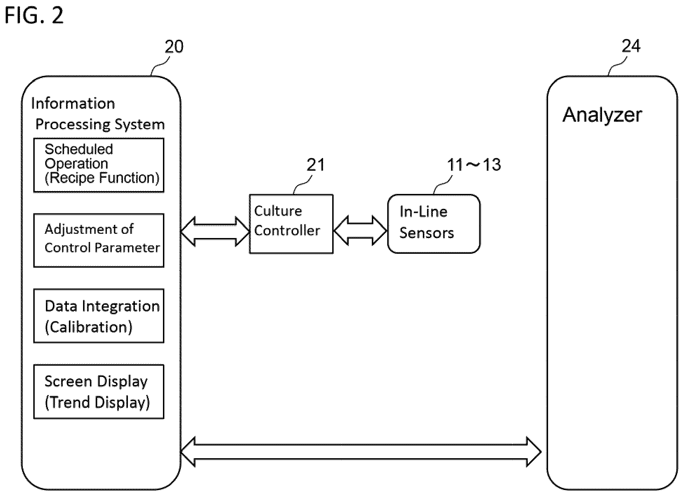

As illustrated in FIG. 2, the information processing system 20 has a recipe function that performs a recipe to perform a scheduled operation of the cell culture system 1. Additionally, a function that adjusts various control parameters when the scheduled operation is performed, a data integration (calibration) function that integrates the obtained data, and a function that adjusts a screen display (trend display) to display the obtained data or the like are provided. The information processing system 20 acquires information from in-line sensors 11 to 13 and the analyzer 24 and controls the system 1 in accordance with the acquired information together with the culture controller 21.

The culture tank 10 is a container into which a medium and cells are supplied to culture the cells. When the culture tank 10 is supplied with the cells and the medium while the temperature, the pH, and the dissolved oxygen (DO) level of the culture fluid are appropriately managed, the cell growth progresses. At a phase where the cells grow to some extent (for example, from two days to three days), the medium is continuously supplied to the culture tank 10, and the cell removal solution (harvest solution) by the amount same as the supplied amount is collected, thus performing the perfusion culture. While the cells are removed by the cell removal device 32 and maintained in the culture tank 10, the harvest solution from which the cells have been removed is discharged to the outside via the flow cell 33 and a pump, so as to allow collecting the harvest solution.

In the perfusion culture, since the supplied amount and the collected amount are the same amount, the culture fluid volume in the culture tank 10 is kept constant. The culture tank 10 is supplied with the medium, glucose, and alkali (Base) via pumps P1 to P3. The information processing system 20 and the culture controller 21 described later control the supplied amount. The supplied amount (volume) of the culture fluid to be supplied can be measured by, for example, a weight scale (scale).

At a start of culture by the perfusion culture, a medium and cells are put in the culture tank 10, and cell growth progresses by appropriately managing the temperature, the pH, and the dissolved oxygen (DO) level of the culture tank 10. At the phase where the cells grow to some extent (two days to three days) in the culture tank 10, the medium is continuously supplied to the culture tank 10, and a cell removal solution (harvest solution) by the same amount as the supplied amount is collected from the culture tank 10 via the flow cell 33 and a pump P5. In the perfusion culture, since the supplied amount to and the collected amount from the culture tank 10 are the same amount, the culture fluid volume in the culture tank 10 is kept constant. Additionally, since the cells remain in the culture tank 10 by a filter action of the cell removal device 32 referred to as tangential flow filtration (TFF) or alternating tangential flow filtration (ATF), even after the perfusion starts, the growth of cells continues in the culture tank 10.

When the cell growth in the culture tank 10 progresses to increase the density of the cells to a certain value, control of the dissolved oxygen (DO) level described above and adjustment of the nutrient become difficult, and a possibility of deterioration of the culture environment increases. Therefore, to reduce the cell density in the culture tank 10 in an appropriate range, means (bleed) that discharges the cells excessively increased from the time point of the growth exceeding the target value to outside the culture tank 10 by removing the culture fluid from the culture tank 10 is taken. That is, the operation of the pump P4 appropriately removes the culture fluid in the culture tank 10 and discharges the culture fluid to the outside.

As described later, since the cells in the culture tank 10 remain in the culture tank 10 by the filter action of the cell removal device 32, even after the perfusion starts, the growth continues in the culture tank 10. The culture controller 21 performs the control for the perfusion culture in accordance with various pieces of information collected by the information processing system 20.

The culture controller 21 monitors the state of the culture fluid in the culture tank 10, performs various controls regarding the perfusion culture step (such as operations of the various pumps, a motor, and the like and temperature control), and controls the supplied amount, the collected amount, the ventilation volume, the perfusion rate amount, and the like in the culture tank 10. Additionally, the culture controller 21 receives the various pieces of information from the information processing system 20, reflects them to control signals, and transmits the situation of control to the information processing system 20.

The information processing system 20 can be constituted by a computer and software readable by the computer. The information processing system 20 can include, in addition to a function of managing a schedule of perfusion culture and a function of integrating the obtained data, a function of designing respective control loops. Note that in the example of FIG. 1, a method in which the culture controller 21 is in charge of individual control of each unit and the information processing system 20 is in charge of upper integration control is described. However, a method in which the information processing system 20 is in charge of a part of or all of the individual controls of the respective units is also possible. The information processing system 20 can also include a display function of a setting screen of process parameters and a trend, a data storage/output function, and the like as a user interface.

The dielectric spectrometer 22 measures the dielectric constant of the culture fluid in accordance with a current flowing between electrodes 12 inserted in the culture fluid and thus measures, for example, the number of cells and the survival rate in the culture fluid. The near-infrared/infrared/Raman spectrometer 23 is a device that can irradiate the culture fluid with various measurement lights (near-infrared light, infrared light, and monochromatic light) by a light emitting and receiving sensor 13 inserted in the culture fluid, measure, for example, a near-infrared spectrum, an infrared spectrum, and a Raman spectrum of light that has passed through or has been reflected by the culture fluid, and measure components of a nutrition and metabolism of cells, such as glucose, lactic acid, ammonia, and various amino acids, in the culture fluid.

The analyzer 24 is a device, such as a high performance liquid chromatographic apparatus (HPLC) or a cell culture analyzer that analyzes the component of the culture fluid sampled from a sampling device 31 at intervals around, for example, once a day, offline (away from the culture tank 10).

In the culture tank 10, the in-line sensors 11 to 13 immersed in the culture fluid to measure the state of the culture fluid during culturing the cells are installed. Additionally, the culture tank 10 also includes a stirring device 15 to stir the culture fluid. The stirring device 15 can operate by driving force from a motor (not illustrated), and the motor can operate, for example, in accordance with the control signal from the culture controller 21.

As one example, the in-line sensors 11 to 13 can include the sensor 11 that measures basic physical/chemical parameters, such as the pH, the dissolved oxygen (DO) level, the temperature, a gas partial pressure (such as a partial pressure of oxygen and a partial pressure of carbon dioxide), an osmotic pressure, a nutrient, a metabolic component, and a target product concentration of the culture fluid and supplies a measurement signal to the culture controller 21, the electrodes 12 of the dielectric spectrometer 22, and the light emitting and receiving sensor 13 of the near-infrared/infrared/Raman spectrometer 23.

In measurement by the dielectric spectrometer 22 and the near-infrared/infrared/Raman spectrometer 23, usually, a calibration model has been preliminarily established using a solution adjusted to be the target component concentration and an absorbance spectrum and a scattering spectrum acquired from, for example, the sensor and the flow cell immersed in the culture tank 10 is converted into the target component concentration in accordance with the calibration model, thus performing the measurement.

The various parameters described above as an example measured during the culture of the cells in the culture tank 10 can be measured by the analyzer 24 by sampling the culture fluid usually around once a day. Note that the cell density in the culture tank 10 can be calculated by capturing an image of the culture fluid after being stained, analyzing the image, and performing counting. An enzyme sensor or the like can be used for the nutrition/metabolic components.

To maintain the environment of the culture tank 10, feedback control to the information processing system 20 is performed on some parameters measured by the in-line sensors 11 to 13 via the culture controller 21. Performing the feedback allows controlling the parameter such that the parameter is in the appropriate control range. Generally, although the control is often performed independent of each parameter by single-input single-output (SISO), the respective parameters interfere with one another in many cases, and therefore stabilization can be achieved by multiple-input multiple-output (MIMO).

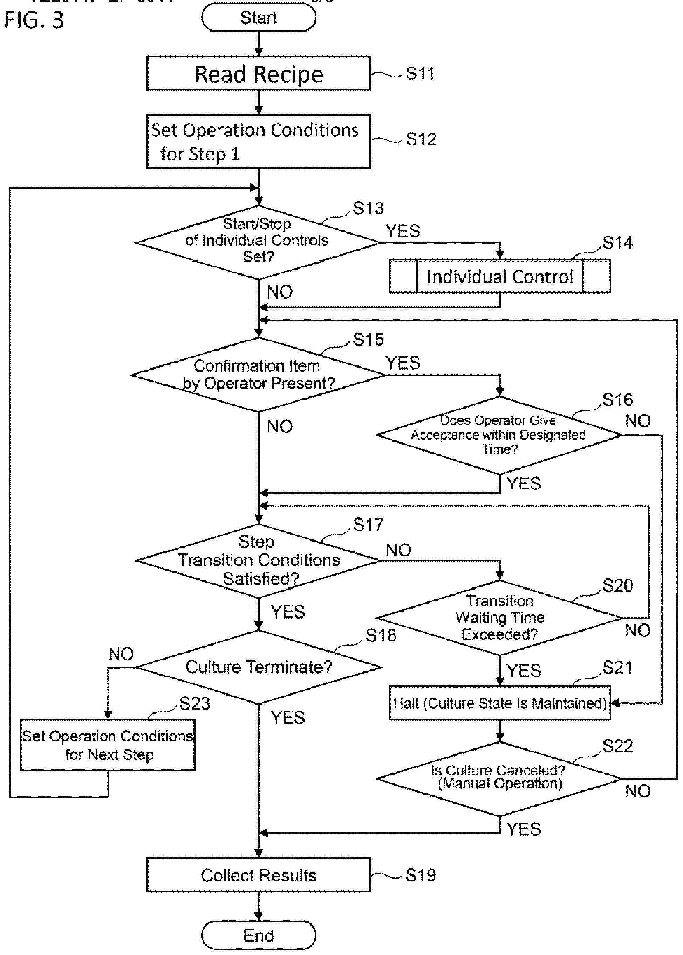

Next, with reference to the flowchart of FIG. 3, the operations in the perfusion culture by the cell culture system 1 is described. Here, the description is given assuming that one batch includes a plurality of steps.

First, in the information processing system 20, software including a recipe file for perfusion culture is read and is installed to the information processing system 20 (Step S11). Instead of external installation, the software may be directly input in the information processing system 20 and may be stored thereafter. The recipe file specifies operation conditions (a culture condition and a control condition) of each of the plurality of steps and transition conditions to the next steps, and in Step S12, the operation conditions for the first step 1 are set (stored).

When the recipe execution operation is performed, the operation conditions for the step described on the recipe are sequentially set, the operation conditions for the "step 1" having the first order are set first and the operation of the perfusion culture starts. Here, when start/stop of individual controls of individual measurement items, such as the temperature and the pH of the culture fluid, are set for the step (YES in Step S13), the sequence of the individual controls described later is performed (Step S14). On the other hand, when the start/stop operations of the individual controls are not set for the individual measurement items (NO in Step S13), the sequence of the individual controls is not performed and the process transitions to Step S15.

In Step S15, it is determined whether an item to be confirmed by an operator is present or not regarding performing the step with the set operation conditions. When the confirmation item is not present, the process transitions to Step S17. When the confirmation item is present (YES), a message regarding the confirmation item is output to the operator via a display (not illustrated) or the like, and it is determined whether the operator accepts the confirmation item within a designated time (Step S16).

In Step S16, when the operator accepts within the designated time (YES in Step S16), the process transitions to Step S17. When the approval is not present (NO), the process transitions to Step S21.

In Step S17, regarding the step currently in execution, it is determined whether the step transition conditions for transition to the next step are satisfied. When the step transition conditions are satisfied (YES), the process transitions to Step S18, and when the step transition conditions are not satisfied, it is waited until the step transition conditions are satisfied until a predetermined waiting time elapses (Step S20). When the step transition conditions are not satisfied during the predetermined waiting time (YES in Step S20), the process transitions to a halt mode, and the step is halted (the culture state is maintained during halt (Step S21)). Afterwards, it is selected by the manual operation by the operator whether to return to the step or cancel the culture (Step S22).

When the step transition conditions are determined to be satisfied in Step S17 (YES), it is determined whether the culture step has terminated in Step S18. When the culture step has not terminated (NO), the process transitions to Step S23, the operation conditions for the next step are set, and hereinafter, the similar procedure is repeated. On the other hand, when the culture step has terminated (YES), data regarding the results of the culture step are collected (Step S13), and the collected information is transmitted to the information processing system 20. Thus, the culture step in one batch terminates.

Note that the culture conditions input to each of the steps can be, for example, changed and added after starting the operation. Additionally, the step in a stage latter than the step in execution can be freely edited. The results of the recipe performed finally are collected after the termination of all of the culture steps.

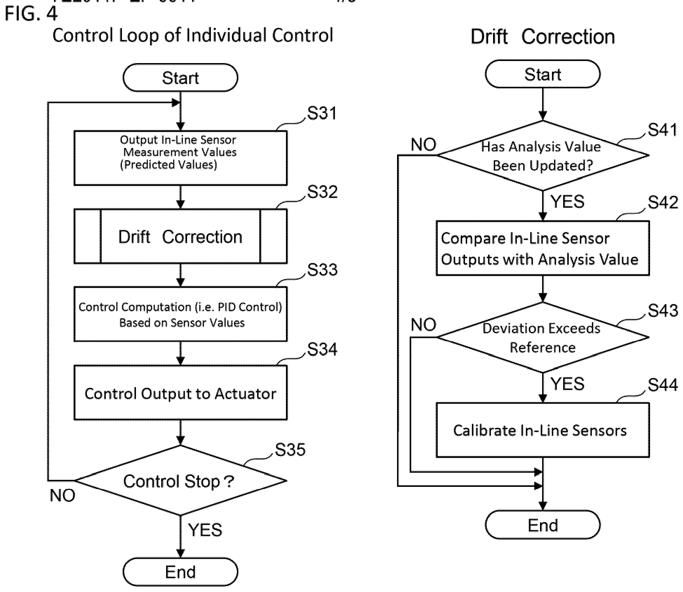

Next, the operations of the above-described individual controls are described with reference to the flowchart of FIG. 4. The flowchart on the left side of FIG. 4 illustrates the entire procedure of the operations of the individual controls, and the flowchart on the right side illustrates a detailed procedure of drift correction among them.

When the individual controls (such as the pH, the dissolved oxygen (DO) level, the temperature, a viable cell density (VCD), the glucose, the liquid amount, and the product concentration) start, measurement values of the respective in-line sensors 11 to 13 (or predicted values) are output to the information processing system 20 and the like (Step S31).

Subsequently, the drift correction of the in-line sensors 11 to 13 is performed (Step S32). Specifically, when the analyzer 24 updates the same parameter (such as the pH, the dissolved oxygen (DO) level, the viable cell density (VCD), the glucose, the liquid amount, and the product concentration) as an offline analysis value (YES in Step S41), the information processing system 20 compares the measurement values of the in-line sensors 11 to 13 with the values of the offline analysis values of the analyzer 24 (Step S42). As the result of the comparison, when the deviations between both are larger than reference values (YES in Step S43), the information processing system 20 calibrates the in-line sensors 11 to 13 in accordance with the deviation amounts (Step S44).

When the drift correction terminates, the information processing system 20 performs control computation (such as PID control) based on the measurement values of the in-line sensors 11 to 13 (Step S33), and outputs the control signals for, for example, the pumps P1 to P5, the motor, and the actuator, to the culture controller 21 (Step S34). The above-described operations are repeated until the operations of the individual controls are stopped by an instruction of control OFF from the information processing system 20 (Step S35).

As described above, in the conventional perfusion culture, in the plurality of batches, the variation in cell growth and various parameters regarding cell growth occur between the batches by being affected by, for example, the manual operation and an error in counting of the number of cells. In contrast, the cell culture system according to this embodiment allows setting the operation conditions for the step and the step transition conditions, which are the conditions for transition to the next step, in the recipe for each of the steps. Therefore, for example, even when the temporally variation occurs in the cell culture, since the transition to the next operation condition is possible in the predetermined cell density and ventilation volume, a repeatable culture profile can be obtained.

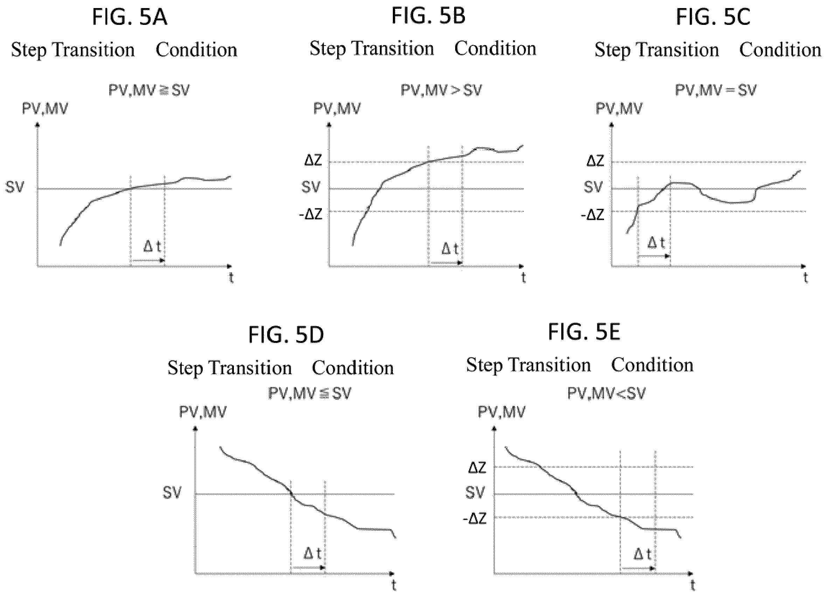

Note that the step transition condition can be, for example, set based on the relationship between measurement values PV or output values MV of the in-line sensors 11 to 13 and a set value SV illustrated in FIG. 5A to FIG. 5D. As one example, as illustrated in FIG. 5A and FIG. 5D, whether an elapsed time Δt after the measurement value PV or the output value MV exceeds or falls below the set value SV exceeds a threshold value Ts can be set as the step transition condition. Alternatively, as illustrated in FIG. 5B, whether the elapsed time Δt after a difference between the measurement value PV or the output value MV and the set value SV exceeds a positive/negative threshold value ±ΔZ exceeds the threshold value Ts can be set as the step transition condition. Alternatively, as illustrated in FIG. 5C and FIG. 5E, whether the elapsed time Δt after the difference between the measurement value PV or the output value MV and the set value SV becomes within the positive/negative threshold value ±ΔZ exceeds the threshold value Ts can be set as the step transition condition.

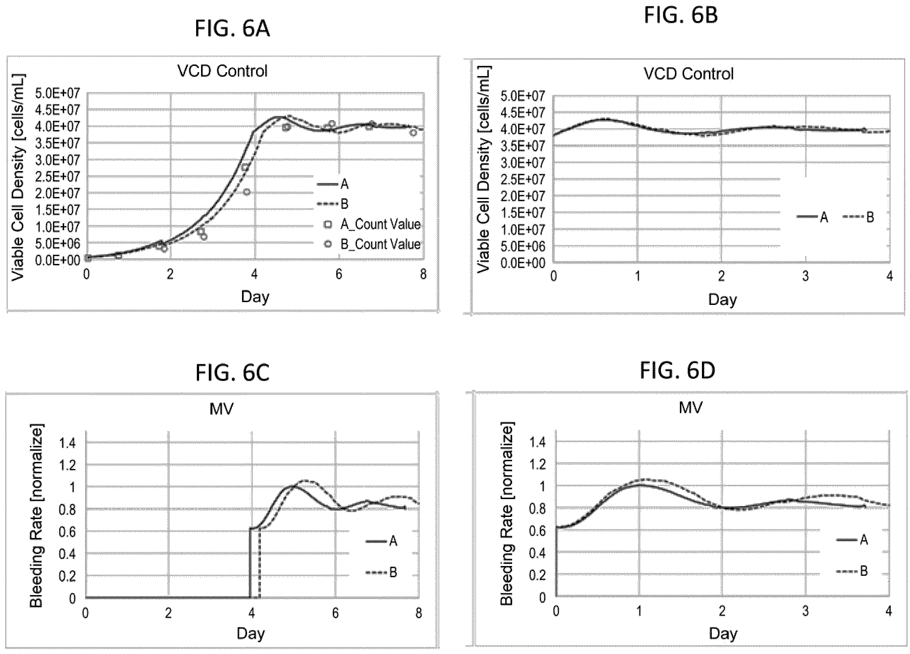

FIG. 6A to FIG. 6D are graphs regarding the viable cell density and the perfusion rate showing the results of calibrating the in-line sensors 11 to 13 in accordance with the results of comparison between the measurement values of the in-line sensors 11 to 13 and the offline analysis values of the analyzer 24. FIG. 6A illustrates the results of comparison between measurement values (A) of the viable cell density by the in-line sensors 11 to 13 and offline analysis values (B) of the analyzer 24. FIG. 6B illustrates the results of comparison between the output values of the in-line sensors 11 to 13 and the offline analysis values of the analyzer 24 regarding the perfusion rate. Here, the set value SV of the viable cell density is set to 4.0 × 10⁷ cells/mL. At the time point where the viable cell density reaches 3.8 × 10⁷ cells/mL, a value as a base for the control output is output, and then the viable cell density is maintained at the set value SV by the feedback control.

FIG. 6C and FIG. 6D illustrate control start points of A and B together. Although a variation in growth or the like occurs between the batches, after the reference value (SV) is reached, a profile exhibiting certain repeatability is obtained both in the measurement value PV and the output value MV.

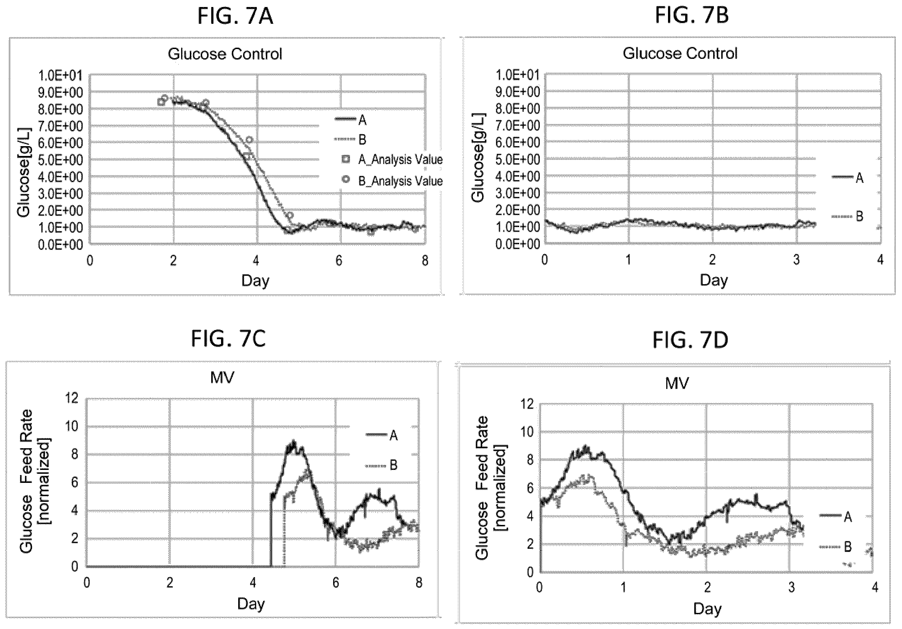

FIG. 7A to FIG. 7D are graphs regarding the glucose concentration and a glucose feed rate showing the results of calibrating the in-line sensors 11 to 13 in accordance with the results of comparison between the measurement values of the in-line sensors 11 to 13 and the offline analysis values of the analyzer 24. FIG. 7A illustrates the result of comparison between the measurement values (A) by the in-line sensors 11 to 13 and the offline analysis values (B) of the analyzer 24 of the glucose concentration. FIG. 7B illustrates the result of comparison between the output values of the in-line sensors 11 to 13 and the offline analysis values of the analyzer 24 regarding the glucose feed rate.

FIG. 7C and FIG. 7D illustrate control start points of A and B together, and similarly to the case of FIG. 6A to FIG. 6D, a profile exhibiting certain repeatability is obtained.

Thus, the real-time observation value is obtained by the use of the in-line sensors 11 to 13, and the flexible transition condition can be set with the recipe function. Thus, the variation between the batches, which is difficult to be handled by the manual operation, can be handled, and the desired culture profile can be obtained.

Additionally, establishing the feedback control system (including combination use with feedforward) using the in-line sensors 11 to 13 and their values allows controlling process parameters affecting the yield and the quality of the final product, such as the number of cells and the glucose concentration, to be within the control range. Since the use of the in-line sensors 11 to 13 increases the number of data points, the establishment of the feedback control system is facilitated. On the other hand, there may be a case where a problem that the drift of the predicted value caused by the variation in the culture environment arises. In this respect, in this embodiment, the use of the offline analysis value allows periodically canceling drift. Additionally, when the parameter affecting the accumulation time of the culture tank 10, such as the perfusion rate, is changed due to the scheduled operation, a process gain in the control loop of the concentration of each of the components, such as glucose, changes, and therefore, for example, proper values of, for example, a proportional gain and an integral time of PID control or the like change. Therefore, as in this embodiment, the configuration that allows setting the control parameter appropriate for each of the steps increases stability.

Additionally, according to this embodiment, the conditions can be sequentially examined, thus allowing the quick process optimization and understanding of the process. That is, while the situation in the culture tank changes by the minute in the batch culture and fed-batch culture, in the consecutive culture method like the perfusion culture, the steady state exists in a certain range (pseudo steady state). To optimize the parameters, since one parameter is basically changed through one-time culture period in non-steady batch culture, considerable man-hour is required.

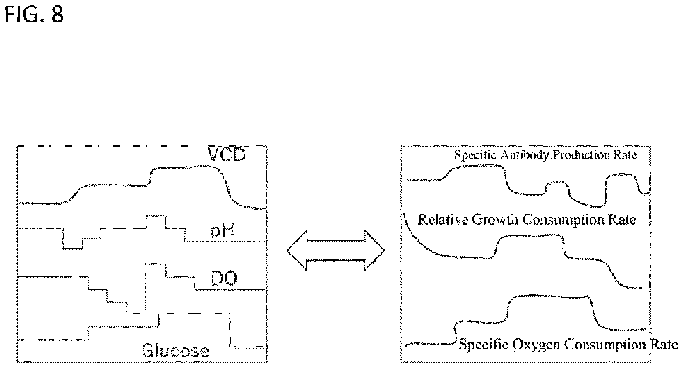

In contrast, the pseudo steady state exists in the perfusion culture, and therefore a plurality of parameters can be optimized during culture. As illustrated in FIG. 8, in this embodiment, after the steady state is established, the various control parameters (such as the pH, the dissolved oxygen level, the temperature, the stirring speed, the glucose concentration, the perfusion rate, and the cell density) can be individually changed at the appropriate timing and influence on each specific rate can be grasped. When sequentially identifying the conditions, dynamic characteristics and stability of control at transition to each of the culture conditions are extremely important to secure repeatability.

The present invention is not limited to the above-described embodiments and various modifications are possible. For example, the above-described embodiments are explained in detail for easy understanding of the present invention, and the present invention is not necessarily limited to the aspect that includes all of the explained configurations. A part of the configuration of one embodiment can be replaced by the configuration of another embodiment, and the configuration of one embodiment can be used with the addition of the configuration of another embodiment. Additionally, for a part of the configurations in the respective embodiments, another configuration can be deleted, added, or replaced.

### DESCRIPTION OF SYMBOLS

- 10: Culture tank
- 11 to 13: In-line sensor
- 15: Stirring device
- P1 to P5: Pump
- 20: Information processing system
- 21: Culture controller
- 22: Dielectric spectrometer
- 23: Near-infrared/infrared/Raman spectrometer
- 24: Analyzer
- 32: Cell removal device
- 33: Flow cell

## Claims

1. A cell culture system comprising:
a culture tank for culturing cells;
an information processing system that processes various pieces of information on the culture of the cells in the culture tank; and
a controller that controls a culture step by the culture tank,
wherein the information processing system being operative to store an operation condition in each of a plurality of steps constituting the culture step of the cells and store a step transition condition for transition to a next step, and set an operation condition for the next step when the step transition condition is satisfied during performing one step.

2. The cell culture system according to claim 1, further comprising
an in-line sensor that is installed in the culture tank and measures a state of a culture fluid in the culture tank.

3. The cell culture system according to claim 2, further comprising
an analyzer that measures the culture fluid sampled from the culture tank offline,
wherein the information processing system compares a measurement value of the in-line sensor with an offline analysis value of the analyzer and calibrates the in-line sensor in accordance with a result of the comparison.

4. The cell culture system according to claim 2 or 3,
wherein the step transition condition is a condition regarding a relationship between a measurement value or an output value of the in-line sensor and a set value.

5. The cell culture system according to claim 1,
wherein when a waiting time to satisfy the step transition condition has elapsed, the controller performs a control to halt a step regarding the step transition condition.

6. The cell culture system according to claim 5,
wherein after performing the control to halt the step, the controller selects cancel of the step or return to the step in accordance with a manual operation by an operator.

7. A cell culture method in a cell culture system including a culture tank for culturing cells, comprising:
storing an operation condition in each of a plurality of steps constituting a culture step of the cells and storing a step transition condition for transition to a next step; and
setting an operation condition for the next step when the step transition condition is satisfied during performing one step.

8. The cell culture method according to claim 7, further comprising
comparing a measurement value of an in-line sensor that is installed in the culture tank and measures a state of a culture fluid in the culture tank with an analysis value of an analyzer that measures the culture fluid sampled from the culture tank offline, and calibrating the in-line sensor in accordance with a result of the comparison.

9. The cell culture method according to claim 8,
wherein the step transition condition is a condition regarding a relationship between the measurement value or an output value of the in-line sensor and a set value.

10. The cell culture method according to claim 7, further comprising
performing a control to halt a step regarding the step transition condition when a waiting time to satisfy the step transition condition has elapsed.

11. The cell culture method according to claim 10, further comprising
selecting cancel of the step or return to the step in accordance with a manual operation by an operator after performing the control to halt the step.
